# EUROPEAN PATENT APPLICATION

(11) **EP 3 639 769 A1**
(43) Date of publication of application: **22.04.2020**
(21) Application number: 19201383.7
(22) Date of filing: 04.10.2019
(51) Int. Cl.: A61B 17/221, A61B 17/3207

(54) **CATHETER**

(30) Priority: 19.10.2018 JP 2018197335
(71) Applicant: Asahi Intecc Co., Ltd., Seto-shi, Aichi 489-0071 (JP)
(72) Inventor: TSUKAMOTO, Toshihiko, Seto-shi, Aichi 489-0071 (JP); NIHONMATSU, Masaaki, Seto-shi, Aichi 489-0071 (JP)
(74) Representative: Winter, Brandl, Fürniss, Hübner, Röss, Kaiser, Polte - Partnerschaft mbB

(57) **Abstract**

Provided is a catheter including a hollow shaft, a mesh member having a tubular shape joined to a distal end side of the hollow shaft and capable of expanding and contracting in radial directions, an expanding and contracting functional portion for expanding and contracting the mesh member, a distal-end fixed member joined to a distal end side of the mesh member, and an aspirator-attachment portion for attachment of an aspirator, the aspirator being for applying negative pressure in the inside of the hollow shaft, the aspirator-attachment portion being provided at a proximal end side of the hollow shaft.

## Description

### CROSS REFERENCE TO RELATED APPLICATION

This application claims the priority based on Japanese patent application No. 2018-197335 filled on October 19, 2018, the entire disclosure of which is incorporated herein by reference.

### TECHNICAL FIELD

The present invention relates to a catheter for removing a foreign matter in a body lumen.

### BACKGROUND ART

Known technologies for treating ischemic cerebral infarction among other cerebral infarctions include, for example, pharmacologic removal technologies and physical removal technologies. As an example of the pharmacologic removal technologies, known is a technology involving administration of a thrombolytic drug, for example, t-PA (tissue plasminogen activator). However, a pharmacologic removal technology alone may disadvantageously require a long time before a drug shows a thrombolytic effect. Treatment prognosis may be improved by reducing a time required for resolving ischemia (a rate of recanalization) in particular when ischemic cerebral infarction is treated. For this reason, in recent years, a pharmacologic removal technology tends to be used in combination with a physical removal technology.

Known physical removal technologies include use of a stent retriever (SR) and thrombus aspiration therapy. For example, an SR technology described in Japanese Patent Application Laid-Open No. 2013-215618 removes a thrombus by interwinding the thrombus using a complex having a distal segment of a mesh structure. Further, a thrombus aspiration therapy described in Japanese Patent Application Laid-Open No. 2015-107301 and Japanese Patent Application Laid-Open No. 2009-66178 capture a thrombus with an unfoldable capturing portion or an elastic deformable portion, and aspirate the captured thrombus with an aspirator attached to the proximal end side of a catheter.

### SUMMARY OF THE INVENTION

### Problems to be Solved by the Invention

Here, a time required for resolving ischemia is desirably reduced in particular for treating ischemic cerebral infarction as described above. In this regard, the technologies described in Japanese Patent Application Laid-Open No. 2013-215618 and Japanese Patent Application Laid-Open No. 2009-66178 require many operating steps: for example, a guide wire is pre-delivered to the distal end of an affected area, and a separate catheter for delivering a complex or an aspiration catheter is then delivered to the affected area; subsequently, a guide wire is withdrawn, as appropriate, and a separate catheter is then used to deliver the complex or the aspiration catheter to the affected area. Therefore, these technologies disadvantageously require a long time before ischemia is resolved. Further, the technology described in Japanese Patent Application Laid-Open No. 2015-107301 does not require a separate catheter, but does require an operating step of expanding a balloon after the balloon is delivered to the distal end of an affected area, similarly resulting in a problem of a long time required before ischemia is resolved.

Moreover, in the technologies described in Japanese Patent Application Laid-Open No. 2013-215618, Japanese Patent Application Laid-Open No. 2015-107301, and Japanese Patent Application Laid-Open No. 2009-66178, a mesh structure for capturing a thrombus, a capturing portion, and an elastic deformable portion are each self-expandable. This may limit a thrombus-capturing effect within a range which can be covered by one expansion. Therefore, there exists a room of improvement in thrombus-capturing performance. It is noted that the above problems are commonly found in general devices for removing a foreign matter in a body lumen such as the vascular system, the lymph gland system, the biliary system, the urinary system, the respiratory tract system, the digestive organ system, the secretory gland, and the reproductive organs.

The present invention is made in order to solve at least a part of the problems as described above. An object of the present invention is to provide a catheter for removing a foreign matter in a body lumen in which a time required to remove the foreign matter can be reduced.

### Means for Solving the Problems

The present invention is made in order to solve at least a part of the above problems, and can be implemented as the following aspects.
(1) According to one aspect of the present invention, a catheter is provided. The catheter includes a a hollow shaft, a mesh member having a tubular shape joined to a distal end side of the hollow shaft and capable of expanding and contracting in the radial directions, an expanding and contracting functional portion for expanding and contracting the mesh member, a distal-end fixed member joined to a distal end side of the mesh member, and an aspirator-attachment portion for attachment of an aspirator, the aspirator-attachment portion being joined to a proximal end side of the hollow shaft.
   According to this configuration, the catheter includes the mesh member having a tubular shape and capable of expanding and contracting in the radial directions at the distal end side of the hollow shaft, and includes the aspirator-attachment portion for applying negative pressure in the inside of the hollow shaft at the proximal end side of the hollow shaft. Consequently, when a guide wire is pre-delivered to the distal end of an affected area, and the catheter having this configuration is then delivered to the affected area, a foreign matter in a body lumen can be captured with the mesh member, and in addition, the captured foreign matter can be aspirated using an aspirator attached to the aspirator-attachment portion. As a result, the catheter having this configuration can reduce a time required to remove a foreign matter as compared with a case in which a separate catheter for delivering a complex and the like and/or a balloon are used.
(2) In the catheter according to the above aspect, the catheter may be configured so that a target matter captured in the inner side of the mesh member is directed toward the aspirator through the inside of the hollow shaft by negative pressure applied in the inside of the hollow shaft when the aspirator attached to the aspirator-attachment portion is activated. According to this configuration, the catheter can direct the target matter (a foreign matter) captured in the inner side of the mesh member toward the aspirator through the inside of the hollow shaft when the aspirator attached to the aspirator-attachment portion is activated. Moreover, an operator can start or stop aspiration of a foreign matter inside the mesh member by turning on or off the aspirator.
(3) In the catheter according to the above aspects, the expanding and contracting functional portion is a core wire having a distal end portion joined to the distal-end fixed member and extending and passing through the insides of the mesh member and the hollow shaft, and the mesh member may expand due to compression along an axis-line direction by the distal-end fixed member and the hollow shaft when the core wire is pulled along the axis-line direction from a proximal end side, and may contract due to extension along the axis-line direction by the distal-end fixed member and the hollow shaft when the core wire is pushed along the axis-line direction from the proximal end side. According to this configuration, the expanding and contracting functional portion is configured as the core wire having the distal end portion joined to the distal-end fixed member and extending and passing through the insides of the mesh member and the hollow shaft. The mesh member expands due to compression along the axis-line direction by the distal-end fixed member and the hollow shaft when the core wire is pulled along the axis-line direction from the proximal end side. Further, the mesh member contracts due to extension along the axis line direction by the distal-end fixed member and the hollow shaft when the core wire is pushed along the axis-line direction from the proximal end side. As described above, an operator can easily control expansion and contraction of the mesh member by pulling and pushing the core wire from the proximal end side. Therefore, the expansion and contraction of the mesh member can also be repeated multiple times according to the intention of the operator. As a result, the catheter having this configuration can improve the foreign-matter capturing capability (the thrombus capturing capability) as compared with the cases in which a self-expandable mesh structure and the like are used.
(4) The catheter according to the above aspects may further include a first film member having a film-like shape, the first film member covering at least a portion of a proximal end side of the mesh member. According to this configuration, the at least a portion of the proximal end portion of the mesh member is covered with the first film member having a film-like shape. This can prevent a foreign matter captured inside the mesh member from leaking outside through the proximal end side of the mesh member.
(5) The catheter according to the above aspects may further include a second film member having a film-like shape, the second film member covering at least a portion of the distal end side of the mesh member. According to this configuration, the at least a portion of the distal end side of the mesh member is covered by the second film member having a film-like shape. This can prevent a foreign matter captured inside the mesh member from leaking outside through the distal end side of the mesh member.
(6) The catheter according to the above aspects may further include an inner hollow shaft having a distal end portion joined to the distal-end fixed member and forming a guide-wire lumen in the inside of the mesh member. According to this configuration, the inner hollow shaft forming the guide-wire lumen in the inner side of the mesh member is further provided. This can prevent the guide wire from exiting outside through a mesh portion of the mesh member, leading to improved catheter handling.

It is noted that the present invention can be implemented according to various aspects. For example, the present invention can be implemented in a aspect of a method of manufacturing a catheter and the like.

### BRIEF DESCRIPTION OF THE DRAWINGS

Fig. 1 shows a schematic partial longitudinal-sectional view of the overall configuration of a catheter according to a first embodiment.
Fig. 2 shows a schematic cross-sectional view along the line A-A in Fig. 1.
Fig. 3 shows a schematic cross-sectional view along the line B-B in Fig. 1.
Fig. 4 shows a schematic partial longitudinal-sectional view of the catheter in a state where a mesh member is expanded.
Figs. 5A to 5E collectively show a diagram illustrating an example of how a thrombus is captured using the catheter according to the present embodiment.
Figs. 6A to 6F collectively show a diagram illustrating an example of how a thrombus is captured using devices of Comparative Example.
Fig. 7 shows a schematic partial longitudinal-sectional view of a distal end side of a catheter according to a second embodiment.
Fig. 8 shows a schematic partial longitudinal-sectional view of a distal end side of a catheter according to a third embodiment.
Fig. 9 shows a schematic partial longitudinal-sectional view of a distal end side of a catheter according to a fourth embodiment.
Fig. 10 shows a schematic partial longitudinal-sectional view of a distal end side of a catheter according to a fifth embodiment.
Fig. 11 shows a schematic partial longitudinal-sectional view of the overall configuration of a catheter according to a sixth embodiment.

### PREFERRED MODE FOR CARRYING OUT THE INVENTION

### First embodiment

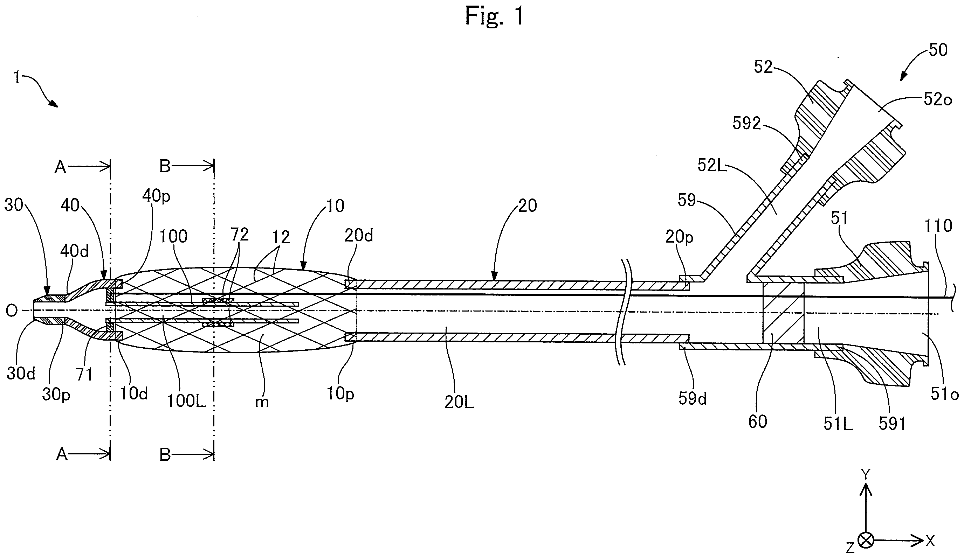

Fig. 1 shows a schematic partial longitudinal-sectional view of the overall configuration of a catheter 1 according to a first embodiment. The catheter 1 is a medical device which can be used for physically removing a thrombus present inside a blood vessel in the treatment of, for example, ischemic cerebral infarction among other cerebral infarctions. It is noted that the catheter 1 can be configured as a device for removing any target matter (a foreign matter such as thrombus) inside a body lumen such as a cerebral blood vessel and other vascular systems, for example, the cardiovascular system; the lymph gland system; the biliary system; the urinary system; the respiratory tract system; the digestive organ system; the secretory gland; and the reproductive organs. The catheter 1 has a mesh member 10, a hollow shaft 20, a distal end tip 30, a distal end shaft 40, a connector 50, and a sealing portion 60. A state where the mesh member 10 is contracted is shown in Fig. 1.

In Fig. 1, an axis line O (a long and short dashed line) represents a longitudinal axis passing through the center of the catheter 1. According to the example shown in Fig. 1, the axis line O coincides with each of the axes passing through the centers of the mesh member 10, the hollow shaft 20, the distal end tip 30, and the distal end shaft 40. However, the axis line O may be different from the central axes of the aforementioned component members. In addition, the XYZ axes which intersect perpendicularly to each other are shown in Fig. 1. The X-axis corresponds to the axis-line direction of the catheter 1, the Y-axis corresponds to the height direction of the catheter 1, and the Z-axis corresponds to the width direction of the catheter 1. The left-hand side (the -X-axis direction) of Fig. 1 is referred to as a "distal end side" of the catheter 1 and each of the component members, and the right-hand side (the +X-axis direction) of Fig. 1 is referred to as a "proximal end side" of the catheter 1 and each of the component members. Further, with regard to the catheter 1 and each of the component members, an end portion located in the distal end side is referred to as a "distal end portion" or simply a "distal end," and an end portion located in the proximal end side is referred to as a "proximal end portion" or simply a "proximal end." The distal end side corresponds to a "distal side" to be inserted into the inside of a body, and the proximal end side corresponds to a "proximal side" to be operated by an operator such as a physician. These also apply to the figures other than Fig. 1 showing overall configurations.

The mesh member 10 has a hollow and subsequently tubular shape having openings at both end portions, i.e., at a distal end portion 10d and a proximal end portion 10p, and is capable of expanding and contracting in the radial directions (in the YZ-axes directions). The mesh member 10 may undergo out-of-plane deformation to expand outwardly in the radial directions as described below, and a thrombus is collected in the inside of the catheter 1 through a mesh opening m of the mesh member 10. The mesh member 10 may have any expansion pressure, outer diameter, and length. The mesh member 10 is arranged between the hollow shaft 20 and the distal end shaft 40 along the direction of the axis line O (the X-axis direction), the distal end shaft 40 serving as the distal-end fixed member. In the present embodiment, the distal end portion 10d of the mesh member 10 is joined to a proximal end portion 40p of the distal end shaft 40. Further, the proximal end portion 10p of the mesh member 10 is joined to a distal end portion 20d of the hollow shaft 20. Joining can be performed by any method. For example, joining can be performed using an adhesive such as an epoxy adhesive, or using silver solder, gold solder, or metal solder such as zinc, Sn-Ag alloy, and Au-Sn alloy.

The mesh member 10 has a plurality of element wires 12. The element wires 12 are braided to form a tubular lattice structure having a mesh opening m. A joining portion at which the element wires 12 are joined to each other is provided at an end portion of the mesh member 10. Any number of joining portions, if any, may be provided at any locations. Each of the element wires 12 may be a solid wire formed of a single element wire, or may be a stranded wire formed of multiple element wires. In a case of a stranded wire, the stranded wire may have any configuration. For example, it may be a twisted wire in which element wires are twisted around a centrally-located core wire (element wire). When a stranded wire is used, the wire diameters and materials of element wires of the stranded wire may be the same or different. For example, one or more of the element wires 12 of the mesh member 10 may be a solid wire(s), and the rest of the element wires 12 may be a stranded wire(s).

Each of the element wires 12 are formed of a metal material or a resin material. As the metal material, for example, stainless steel such as SUS304, nickel-titanium alloy, cobalt-chromium alloy, and the like may be used. Other than these, radiopaque materials such as gold, platinum, tungsten, and alloys including these elements may be preferably used for a material(s) of the element wires 12 to improve visibility of the mesh member 10 under radioscopy. As the resin material, for example, polyamide, polyester, polyacrylate, polyetheretherketone, and the like may be used. The same material may be used for all of the element wires 12 of the mesh member 10, or different materials may be used for one or more of the element wires 12. When different materials are used, combinations of different metal materials, combinations of different resin materials, combinations of a metal material and a resin material, and the like may be used.

The hollow shaft 20 is an elongated member extending along the axis line O. The hollow shaft 20 has a hollow and subsequently tubular shape having openings at both end portions, i.e., at the distal end portion 20d and a proximal end portion 20p. The hollow shaft 20 has an inner cavity 20L in the inside thereof. The inner cavity 20L serves as a guide-wire lumen through which a guide wire is to be inserted into the catheter 1, and also serves as a thrombus-collecting lumen through which a thrombus captured by the mesh member 10 is directed to an aspirator. The hollow shaft 20 may have any outer diameter and length, and the inner cavity 20L may have any inner diameter. According to the present embodiment, the proximal end portion 10p of the mesh member 10 is joined to the outer peripheral surface of the distal end portion 20d of the hollow shaft 20. Further, the connector 50 is joined to the outer peripheral surface of the proximal end portion 20p of the hollow shaft 20. The hollow shaft 20 may have a configuration in which a plurality of shafts are connected. In this case, the plurality of shafts may be formed of the same material to each other, or may be formed of different materials to each other.

The hollow shaft 20 preferably has antithrombogenicity, flexibility, and biocompatibility, and may be formed of a resin material or a metal material. As the resin material, for example, polyamide resin, polyolefin resin, polyester resin, polyurethane resin, silicone resin, fluororesin, and the like may be used. As the metal material, for example, stainless steel such as SUS304, nickel-titanium alloy, cobalt-chromium alloy, and the like may be used. Other than these, radiopaque materials such as gold, platinum, tungsten, and alloys including these elements may be preferably used as a material of the hollow shaft 20 to improve visibility of the hollow shaft 20 under radioscopy.

The distal end tip 30 is arranged at the distal end of the catheter 1, and intended to advance through a blood vessel prior to other members. The distal end tip 30 has a hollow shape having openings at both end portions, i.e., at a distal end portion 30d and a proximal end portion 30p. The distal end tip 30 has an outer shape tapered toward the distal end side from the proximal end side in order to facilitate smooth advance of the catheter 1 through a blood vessel. The distal end tip 30 may have any outer diameter and length. According to the present embodiment, the proximal end portion 30p of the distal end tip 30 is joined to a distal end portion 40d of the distal end shaft 40. The distal end tip 30 preferably has flexibility, and thus may be formed of, for example, a resin material such as polyurethane and polyurethane elastomer. It is noted that the same material as the hollow shaft 20 may also be selected to improve visibility of the distal end tip under radioscopy.

The distal-end fixed member serves to fix the distal end portion of the mesh member, and in the first embodiment, corresponds to the distal end shaft 40 arranged between the mesh member 10 and the distal end tip 30. The distal end shaft 40 has a hollow shape having openings at both end portions, i.e., at the distal end portion 40d and the proximal end portion 40p, and has an outer shape tapered toward the distal end side from the proximal end side as in the distal end tip 30. The distal end shaft 40 may have any outer diameter and length. According to the present embodiment, the distal end portion 40d of the distal end shaft 40 is joined to the proximal end portion 30p of the distal end tip 30. Further, the distal end portion 10d of the mesh member 10 is joined to the outer peripheral surface of the proximal end portion 40p of the distal end shaft 40. As in the hollow shaft 20, the distal end shaft 40 preferably has antithrombogenicity, flexibility, and biocompatibility, and may be formed of a resin material or a metal material.

The connector 50 as an aspirator-attachment portion is arranged at the proximal end side of the catheter 1, and will be grasped by an operator. The connector 50 includes a branched portion 59 which is hollow and bifurcate, and vane portions 51, 52. An inner cavity 51L leading to a first proximal end portion 591 in the branched portion 59 serves as as a guide-wire lumen through which a guide wire is to be inserted into the catheter 1. Further, an inner cavity 52L leading to a second proximal end portion 592 in the connector 50 serves as a thrombus lumen through which a thrombus carried via the hollow shaft 20 is directed to an aspirator. The branched portion 59 may have any shape, outer diameter, and length, and the inner cavity 51L and the inner cavity 52L may have any inner diameter. According to the present embodiment, the proximal end portion 20p of the hollow shaft 20 is joined to the inner peripheral surface of a distal end portion 59d of the branched portion 59. Further, the vane portion 51 is joined to the outer peripheral surface of the first proximal end portion 591 of the distal end portion 59d, and the vane portion 52 is joined to the outer peripheral surface of the second proximal end portion 592.

The vane portion 51 has a hollow shape having openings at both end portions, i.e., at a distal end portion and a proximal end portion. A core wire 110 described below is exposed through an opening 51o at a proximal end side leading to the inner cavity 51L of the vane portion 51. As in the vane portion 51, the vane portion 52 has a hollow shape having openings at both end portions, i.e., at a distal end portion and a proximal end portion. An opening 52o at a proximal end side leading to the inner cavity 52L of the vane portion 52 is configured so that an aspirator can be attached. The vane portions 51, 52 may have any shapes. The vane portions 51, 52 may be omitted when appropriate. The branched portion 59, the vane portion 51, and the vane portion 52 may be formed of a resin material(s) such as polyamide, polypropylene, polycarbonate, polyacetal, and polyether sulfone. It is noted that there is no particular limitation for a method of driving the core wire, but for example, the core wire may be driven back and forth by converting rotational movement resulted from rotational operations of a member attached to the catheter into linear movement, or may be driven back and forth using an an actuator. Moreover, the core wire may also be driven in a state where the core wire is exposed outside the hollow shaft, or in a state where the core wire is not exposed outside the hollow shaft (a state where the core wire is contained inside the hollow shaft).

The sealing portion 60 is arranged in the inner cavity 51L of the branched portion 59. The sealing portion 60 can allow a guide wire and the core wire 110 to be inserted therethrough, and seals the inner cavity 51L to maintain airtightness of the inside of the hollow shaft 20. The sealing portion 60 may be configured, for example, as a valve body. It is noted that the sealing portion 60 does not always need to seal the inner cavity 51L, and may have a structure which can seal the inner cavity 51L at least when an aspirator is activated (when negative pressure is applied in the inside of the hollow shaft 20).

An inner hollow shaft 100 is an elongated member extending along the axis line O. The inner hollow shaft 100 has a hollow and substantially tubular shape having openings at both end portions, i.e., at a distal end portion and a proximal end portion and having an inner cavity 100L in the inside thereof. The inner cavity 100L, in the inside of the mesh member 10, serves as a guide-wire lumen through which a guide wire is to be inserted. Preferably, the inner hollow shaft 100 has an outer diameter smaller than each of the inner diameters of the mesh member 10, the hollow shaft 20, and the distal end shaft 40 described above. However, it may have any appropriate outer diameter and length. According to the present embodiment, the distal end portion of the inner hollow shaft 100 is joined to the proximal end portion 40p of the distal end shaft 40 through a joining portion 71 described below. As in the hollow shaft 20, the inner hollow shaft 100 preferably has antithrombogenicity, flexibility, and biocompatibility, and may be formed of a resin material or a metal material.

The core wire 110 is an elongated solid member which can serve as an expanding and contracting functional portion for expanding and contracting the mesh member 10. The core wire 110 may have any outer diameter and length. According to the present embodiment, the distal end portion of the core wire 110 is joined to the proximal end portion 40p of the distal end shaft 40 through the joining portion 71 described below. Further, the proximal end portion of the core wire 110 extends through each of the insides of the mesh member 10, the hollow shaft 20, and the connector 50, and is exposed outside through the opening 51o of the vane portion 51. The core wire 110 preferably has high tensile strength and stiffness so that a force enough to expand and contract the mesh member 10 can be applied while breakage of the core wire 110 can be prevented. To this end, the core wire 110 may be formed of, for example, a metal material such as stainless steel such as SUS304, nickel titanium alloy, cobalt-chromium alloy, and tungsten alloy.

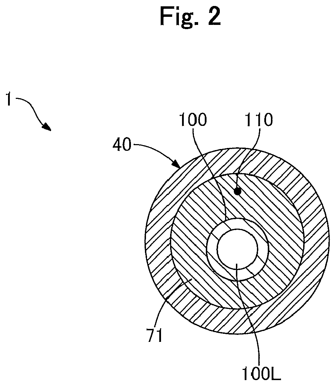

Fig. 2 shows a schematic cross-sectional view along the line A-A in Fig. 1. Fig. 2 shows a cross-section of the catheter 1 in the vicinity of the proximal end portion 40p of the distal end shaft 40. As shown in the figure, the distal end shaft 40, the inner hollow shaft 100, and the core wire 110 are fixed together in an integrated manner through the joining portion 71 inside the proximal end portion 40p of the distal end shaft 40. Further, a guide-wire lumen (the inner cavity 100L) through which the distal end tip 30 and the hollow shaft 20 are to be inserted and passed is formed in the inner side the inner hollow shaft 100. The joining portion 71 may be formed of, for example, an adhesive like an epoxy-based resin, polyamide resin, polyolefin resin, polyester resin, polyurethane resin, silicon resin, fluororesin, polyamide elastomer, polyolefin elastomer, polyester elastomer, and polyurethane elastomer.

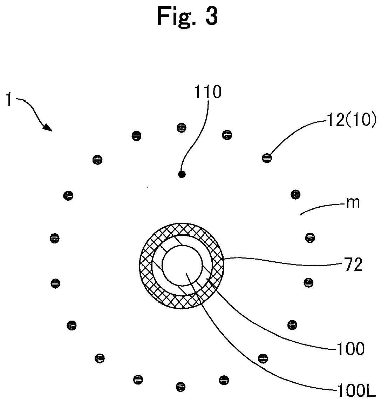

Fig. 3 shows a schematic cross-sectional view along the line B-B in Fig. 1. Fig. 3 shows a cross-section of the catheter 1 at the substantially middle portion of the mesh member 10. As shown in the figure, the mesh member 10 includes the element wires 12 braided so as to have a mesh opening m, and has a space in the inner side of the mesh opening m. Further, the inner hollow shaft 100 and the core wire 110 are arranged in the inner side (space) of the mesh member 10. The guide-wire lumen (the inner cavity 100L) through which the distal end tip 30 and the hollow shaft 20 are to be inserted and passed is formed in the inner side of the inner hollow shaft 100. Further, a marker 72 is joined to the outer peripheral surface of the inner hollow shaft 100.

The marker 72 serves as a marking for indicating the location of the mesh member 10. The marker 72 may be formed of a resin or metal material having radiopacity. For example, when a resin material is used, the marker 72 may be formed by mixing polyamide resin, polyolefin resin, polyester resin, polyurethane resin, silicon resin, fluororesin, and the like with a radiopaque material such as bismuth trioxide, tungsten, and barium sulfate. For example, when a metal material is used, the marker 72 may be formed of gold, platinum, tungsten, which are radiopaque, and an alloy (for example, a platinum-nickel alloy) including these elements, and the like.

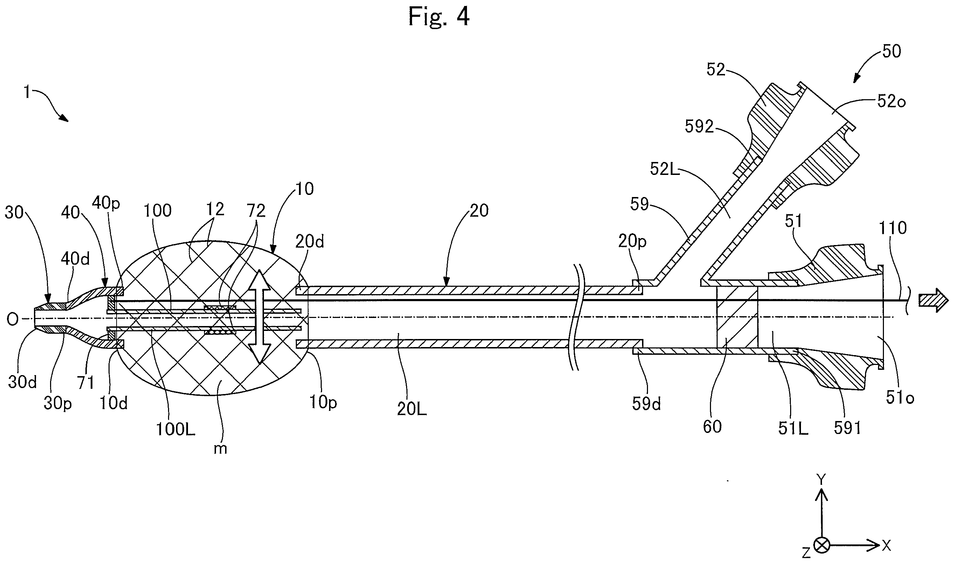

Fig. 4 shows a schematic partial longitudinal-sectional view of the catheter 1 in a state where the mesh member 10 is expanded. In the catheter 1 according to the present embodiment, the following procedures may be used to expand or contract the mesh member 10 (expansion/contraction). In order to expand the mesh member 10 in a contracted state, an operator pulls the core wire 110 exposed through the connector 50 along the direction of the axis line O (the +X-axis direction, Fig. 4: the direction indicated by a hatched arrow) from the proximal end side. Here, the core wire 110 is fixed to the distal end shaft 40 through the joining portion 71. Therefore, the mesh member 10 is compressed along the direction of the axis line O by the distal end shaft 40 and the hollow shaft 20 when the core wire 110 is pulled. This causes the mesh member 10 to expand in the radial directions (the YZ-axes directions, Fig. 4: the directions indicated by a void arrow).

On the other hand, in order to contract the mesh member 10 in an expanded state, an operator pushes the core wire 110 exposed through the connector 50 inwardly along the direction of the axis line O (the -X-axis direction) from the proximal end side. As described above, the core wire 110 is fixed to the distal end shaft 40 through the joining portion 71. Therefore, the mesh member 10 is extended along the direction of the axis line O by the distal end shaft 40 and the hollow shaft 20 when the core wire 110 is pushed inwardly. This causes the mesh member 10 to contract in the radial directions (the YZ-axes directions).

It is noted that the mesh member 10 can be expanded and contracted differently from the aforementioned method. Specifically, an operator may push the connector 50 (and the hollow shaft 20 joined to the connector 50) inwardly along the direction of the axis line O (the -X-axis direction) while supporting the core wire 110 exposed through the connector 50. Consequently, the mesh member 10 is compressed by the distal end shaft 40 and the hollow shaft 20 along the direction of the axis line O, leading to radial expansion. Similarly, an operator may pull the connector 50 along the direction of the of axis line O (the +X-axis direction) while supporting the core wire 110 exposed through the connector 50. Consequently, the mesh member 10 is extended by the distal end shaft 40 and the hollow shaft 20 along the direction of the axis line O, leading to radial contraction.

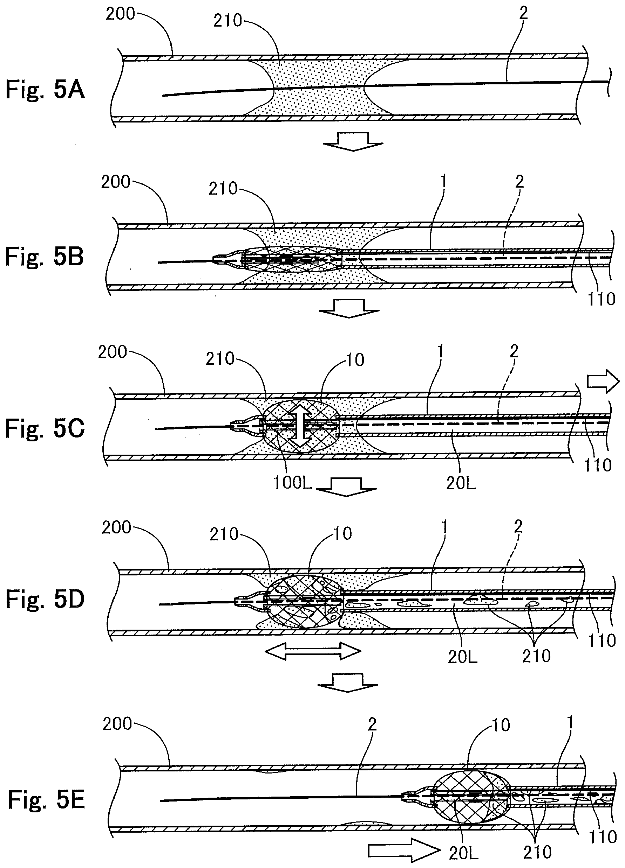

Figs. 5 shows diagrams illustrating an example of how a thrombus is captured using the catheter 1 according to the present embodiment. In Fig. 5, a blood vessel 200 is illustrated as an example of a body lumen while a thrombus 210 is illustrated as an example of a foreign matter. As illustrated in the diagrams, an affected area is formed in which the blood vessel 200 is occluded with the thrombus 210. Fig. 5A shows a state where a guide wire 2 is delivered. Fig. 5B shows a state where the catheter 1 according to the present embodiment is delivered. Fig. 5C shows a state where the mesh member 10 is expanded. Fig. 5D shows a state where the thrombus 210 is captured and collected. Fig. 5E shows a state where the catheter 1 is being withdrawn.

As shown in Fig. 5A, an operator first delivers the guide wire 2 so that the distal end portion of the guide wire 2 is positioned in the distal end side of the thrombus 210 (the affected area). Then, an operator inserts the catheter 1 from the proximal end side of the guide wire 2, allowing the guide wire 2 to be inserted and passed through a guide-wire lumen of the catheter 1 as shown in Fig. 5B. While maintaining that state, the operator pushes the catheter 1 along the guide wire 2, and continues to deliver the catheter 1 until the mesh member 10 reaches the location of the thrombus 210. During the delivery, the mesh member 10 of the catheter 1 remains contracted.

After the mesh member 10 reaches the location of the thrombus 210, the operator pulls the core wire 110 along the axis-line direction, causing the mesh member 10 to expand as shown in Fig. 5C. In this way, the thrombus 210, which is coagulated, can be scraped off and removed from the inner wall of the blood vessel 200 using the the element wires 12 of the mesh member 10 in the expanded state. Further, the thrombus 210 scraped off may be collected in the inner side of the mesh member 10 through the mesh opening m. Subsequently, the operator activates an aspirator attached to the connector 50 as shown in Fig. 5D. Then, the thrombus 210 collected in the inner side of the mesh member 10 is directed to the aspirator, and aspirated and removed through a thrombus-collecting lumen (the inner cavity 20L) of the hollow shaft 20.

The operator may repeat the combination of moving the location of the mesh member 10 along the axis-line direction as shown in Fig. 5B, expanding and contracting the mesh member 10 as shown in Fig. 5C, and starting and stopping the aspirator as shown in Fig. 5D, if desired. Thereby, the thrombus 210 formed in the blood vessel 200 can be entirely captured and collected as shown in Fig. 5E. After the collection of the thrombus 210 is completed, the operator may withdraw the catheter 1 as shown in Fig. 5E. According to the catheter 1 of the present embodiment, the thrombus 210 collected in the inner side of the mesh member 10 is aspirated with an aspirator. This can prevent leakage of the thrombus 210 through the mesh opening m of the mesh member 10 when the catheter is withdrawn.

It is noted that the example shown in Fig. 5 illustrates a case in which the mesh member 10 is stopped and expanded at the location of the thrombus 210. However, the mesh member 10 may be stopped and expanded at any location. For example, an operator may deliver the mesh member 10 to the distal end side of the thrombus 210, and then expand the mesh member 10. In that case, the operator can scrape off the thrombus 210 while pulling back the catheter 1 from the distal end side of the thrombus 210 to the proximal end side. For example, the operator may deliver the mesh member 10 to the proximal end side of the thrombus 210, and then expand the mesh member 10. In that case, the operator can scrape off the thrombus 210 while pushing the catheter 1 inwardly to the distal end side of the thrombus 210 from the proximal end side. It is noted that another catheter into which the catheter 1 is movably received may be used in order to deliver the catheter 1 to the thrombus 210, or in order to remove the catheter 1 out of the body.

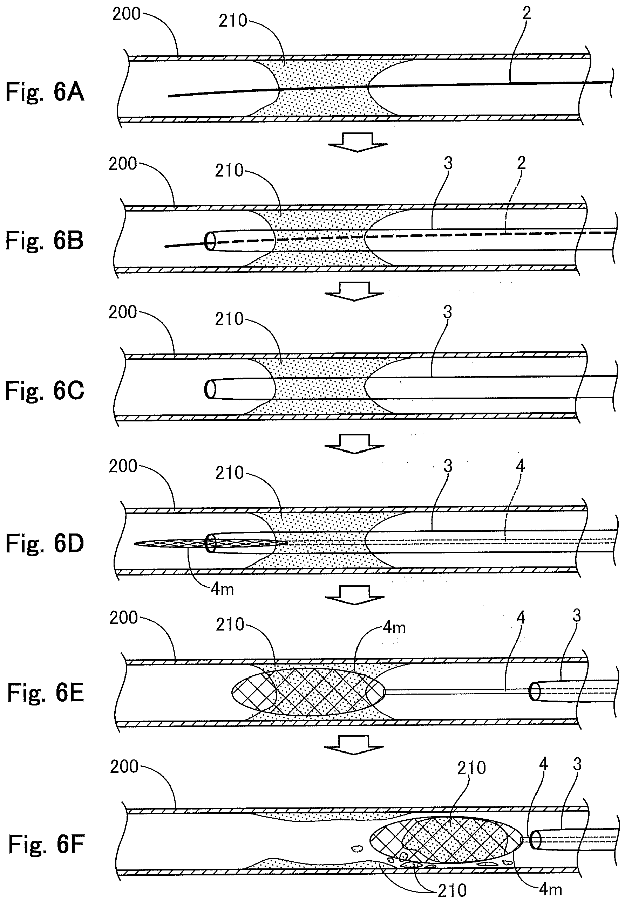

Figs. 6 shows diagrams illustrating an example of how a thrombus is captured using devices of Comparative Example. In Fig. 6, a delivery catheter 3 for delivering a complex 4 and the complex 4 including a distal end portion having a self-extendable mesh structure 4m are illustrated as devices of Comparative Example. Fig. 6A shows a state where a guide wire 2 is delivered. Fig. 6B shows a state where the delivery catheter 3 is delivered. Fig. 6C shows a state where a guide wire 2 is being withdrawn. Fig. 6D shows a state where the complex 4 is delivered. Fig. 6E shows a state where the thrombus 210 is captured. Fig. 6F shows a state where the complex 4 is being withdrawn.

Also, in the case of Comparative Example, an operator first delivers the guide wire 2 to the distal end side of the thrombus 210 as shown in Fig. 6A. Next, the operator inserts the delivery catheter 3 from the proximal end side of the guide wire 2 as shown in Fig. 6B. While maintaining that state, the operator pushes the delivery catheter 3 inwardly along the guide wire 2, and continues to deliver the delivery catheter 3 until an opening at the distal end side reaches the distal end side of the thrombus 210. Next, the operator withdraws the guide wire 2 as shown in Fig. 6C.

Subsequently, the operator inserts the complex 4 from the proximal end side of the delivery catheter 3, and pushes the complex 4 inwardly along an inner cavity of the delivery catheter 3 as shown in Fig. 6D. The mesh structure 4m of the complex 4 is a self-extendable as described above. Therefore, the mesh structure 4m of the complex 4 undergoes expansion when exposed from an opening at the distal end side of the delivery catheter 3 as shown in Fig. 6E. While maintaining that state, the operator pulls the complex 4 back from the distal end side of the thrombus 210 toward the proximal end side as shown in Fig. 6F. Thereby, the thrombus 210 which is coagulated can be scraped off and removed from the inner wall of the blood vessel 200 using element wires of the mesh structure 4m.

As described above, the thrombus 210 can also be scraped off and removed from the inner wall of the blood vessel 200 even in the case of Comparative Example. However, in the case of Comparative Example, additional steps are required as shown in Fig. 6, costing more time to remove the thrombus 210. Further, in the case of Comparative Example, the mesh structure 4m can not be expanded and expanded multiple times, resulting in potential leftovers of the thrombus 210 as shown in Fig. 6F. Therefore, the foreign-matter capturing capability is inferior. Moreover, in the case of Comparative Example, the thrombus 210 which has been scraped off can not be aspirated, resulting in a risk where the thrombus 210 collected in the inner side of the mesh structure 4m may be leaked outside the inner side of the mesh structure 4m as shown in Fig. 6F

As described in the above, the catheter 1 according to the present embodiment includes the mesh member 10 having a tubular shape and capable of expanding and contracting in the radial directions (the YZ directions) at the distal end side of the hollow shaft 20, and the connector 50 (the aspirator-attachment portion) for applying negative pressure in the inside of the hollow shaft 20 at the proximal end side of the hollow shaft 20. Therefore, when the guide wire 2 is pre-delivered to the distal end of the thrombus 210 (the affected area), and the catheter 1 according to the present embodiment is then delivered to the thrombus 210, the thrombus 210 (a foreign matter in a body lumen) in the blood vessel 200 can be captured with the mesh member 10, and in addition, the thrombus 210 captured can be aspirated using an aspirator attached to the connector 50. As a result, the catheter 1 according to the present embodiment can reduce a time required to remove a foreign matter as compared with a case where the catheter 3 provided separately is used to deliver a complex and the like (as illustrated in Fig. 6) and a case where a balloon provided separately is used.

Further, as illustrated in Fig. 5D, the catheter 1 according to the present embodiment can direct the thrombus 210 (a target matter, a foreign matter) captured in the inner side of the mesh member 10 to an aspirator via the inner cavity 20L (inside) of the hollow shaft 20 when the aspirator attached to the connector 50 (the aspirator-attachment portion) is activated to apply negative pressure in the inside of the hollow shaft. This enables an operator to start or stop aspiration of the foreign matter inside the mesh member by turning on or off the aspirator.

Furthermore, the expanding and contracting functional portion in the catheter 1 according to the present embodiment is configured to include a distal end portion joined to the distal end shaft 40 (the distal-end fixed member), and the core wire 110 extending and passing through the insides of the mesh member 10 and the hollow shaft 20. The mesh member 10 is compressed by the distal end shaft 40 and the hollow shaft 20 along the direction of the axis line O to undergo expansion when the core wire 110 is pulled along the direction of the axis line O from the proximal end side. Meanwhile, the mesh member 10 is extended by the distal end shaft 40 and the hollow shaft 20 along the direction of the axis line O to undergo contraction when the core wire 110 is pushed along the direction of the axis line O from the proximal end side. In this way, an operator can easily perform expansion and contraction of the mesh member 10 by pulling and pushing the core wire 110 from the proximal end side, respectively. This also enables the operator to expand and contract the mesh member 10 multiple time according to her/his intention. As a result, the catheter 1 according to the present embodiment can improve the foreign-matter capturing capability (the thrombus capturing capability) as compared with the case where the self-expandable mesh structure 4m and the like are used (as illustrated in Fig. 6).

In addition, the catheter 1 according to the present embodiment further include the inner hollow shaft 100 which forms a guide-wire lumen in the inner side of the mesh member 10. This configuration can prevent the guide wire 2 from exiting outside from the mesh opening m (a mesh portion) of the mesh member 10, leading to improved catheter handling.

### Second embodiment

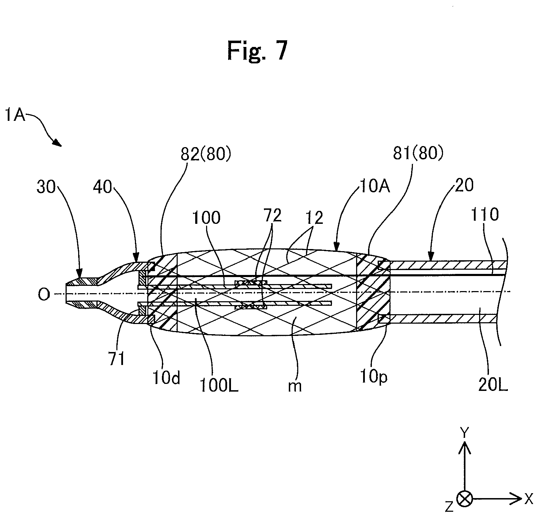

Fig. 7 shows a schematic partial longitudinal-sectional view of a distal end side of a catheter 1A according to a second embodiment. It is noted that the XYZ axes indicated in the figures in Fig. 7 and hereafter correspond to the XYZ axes of Fig. 1. In addition to the configuration described with reference to the first embodiment, the catheter 1A according to the second embodiment further includes a film member 80 for preventing leakage of a thrombus captured in the inner side a mesh member 10A.

The film member 80 includes a first film member 81 and the second film member 82. The first film member 81 has a thin-film like shape, and configured so as to cover a mesh member 10A (in other words, to burry the opening m between the element wires 12) from the proximal end portion 10p of the mesh member 10A to at least a portion of the proximal end side of the mesh member 10A. Similarly, the second film member 82 has a thin-film like shape, and configured so as to cover the mesh member 10A (in other words, to burry the opening m between the element wires 12) from a distal end portion 10d of the mesh member 10A to at least a portion of a distal end side of the mesh member 10A. The first and second film member 81, 82 may have any length in the direction of an axis line O.

The first and the second film members 81, 82 are formed of an elastic material(s) which can stretch and shrink as the mesh member 10A expands and contracts. The first and second film members 81, 82 can be formed of, for example, polyethylene, polyurethane, polyamide, polyamide elastomer, polyolefin, polyolefin elastomer, polyester, polyester elastomer, and the like. The first and second film members 81, 82 may be formed by joining a thin film to the element wires 12, or may be formed by the dip method.

The catheter 1A according to the second embodiment as described above can also demonstrate similar effects as the aforementioned first embodiment does. Moreover, in the catheter 1A according to the second embodiment, at least a portion of the proximal end side of the mesh member 10A is covered with the first film member 81 having a film-like shape, and at least a portion of the distal end side is covered with the second film member 82 having a film-like shape. This configuration can prevent a thrombus (a foreign matter) captured inside the mesh member 10A from leaking outside through the proximal end side and the distal end side of the mesh member 10A.

### Third embodiment

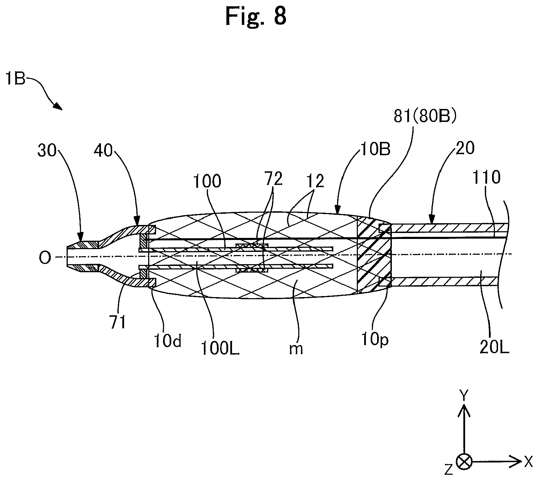

Fig. 8 shows a schematic partial longitudinal-sectional view of a distal end side of a catheter 1B according to a third embodiment. The catheter 1B according to the third embodiment includes a film member 80B instead of the film member 80 described in the second embodiment. The film member 80B includes the first film member 81, but does not include the second film member 82. The first film member 81 is configured as described in the second embodiment. The catheter 1B according to the third embodiment as described above can also demonstrate similar effects as the aforementioned first embodiment does. Further, in the catheter 1B according to the third embodiment, at least portion of a proximal end side of a mesh member 10B is covered with the first film member 81 having a film-like shape. This configuration can prevent a thrombus (a foreign matter) captured inside the mesh member 10B from leaking outside through the proximal end side of the mesh member 10B. It is noted that a film member including the second film member 82 but not the first film member 81 may be used instead of the film member 80B.

### Fourth embodiment

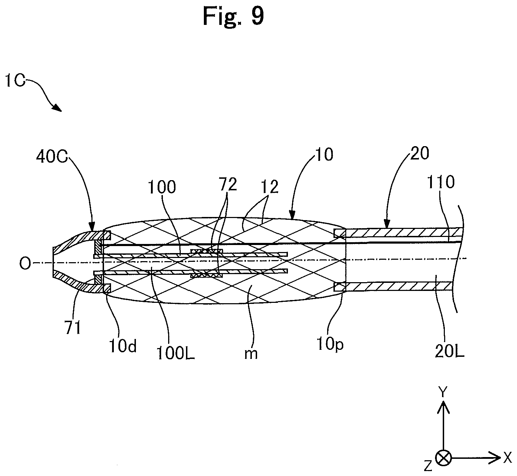

Fig. 9 shows a schematic partial longitudinal-sectional view of a distal end side of a catheter 1C according to a fourth embodiment. The catheter 1C according to the fourth embodiment does not include the distal end tip 30 provided in the configuration described in the first embodiment. In the catheter 1C according to the fourth embodiment, a distal end shaft 40C serves as a distal-end fixed member, and is arranged at the distal end of the catheter 1C, and thus will advance through a blood vessel prior to other members. For this reason, the distal end shaft 40C preferably has plasticity, and is preferably formed of, for example, a resin material such as polyurethane and polyurethane elastomer.

It is noted that a configuration which does not include the distal end tip 30 is exemplified in the fourth embodiment, but the catheter 1C may include a distal end tip 30C, but not the distal end shaft 40. In this case, the distal end tip 30C serves as a distal-end fixed member, and the mesh member 10, the inner hollow shaft 100, and the core wire 110 are joined to the distal end tip 30C (Fig. 2). The catheter 1C according to the fourth embodiment as described above can also demonstrate similar effects as the aforementioned first embodiment does. Moreover, in the catheter 1C according to the fourth embodiment, either one of the distal end tip 30 or the distal end shaft 40 may be omitted to simplify the configuration of the catheter 1C.

### Fifth embodiment

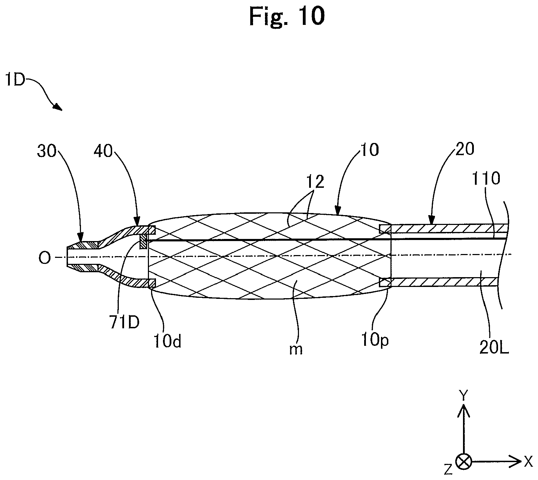

Fig. 10 shows a schematic partial longitudinal-sectional view of a distal end side of a catheter 1D according to a fifth embodiment. The catheter 1D according to the fifth embodiment does not include the inner hollow shaft 100 provided in the configuration described in the first embodiment. In the catheter 1D according to the fifth embodiment, a joining portion 71D joins the core wire 110 with the distal end shaft 40 in the inside of the proximal end portion 40p of the distal end shaft 40. A similar adhesive as used for the joining portion 71 may be used for the joining portion 71D. The same adhesive may be used for both the joining portion 71D and the joining portion 71, or different adhesives may be used. The catheter 1D according to the fifth embodiment as described above can also demonstrate similar effects as the aforementioned first embodiment does.

### Sixth embodiment

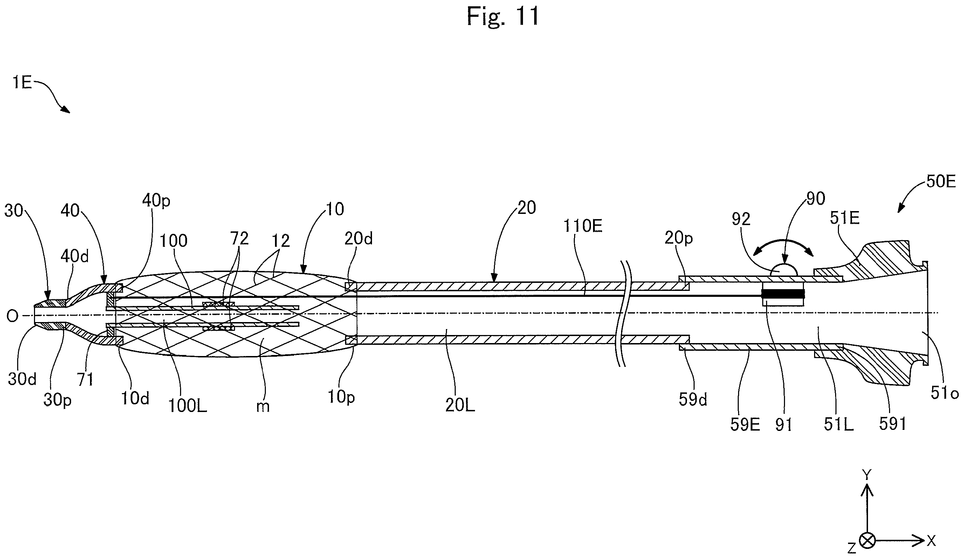

Fig. 11 shows a schematic partial longitudinal-sectional view of the overall configuration of a catheter 1E according to a sixth embodiment. The catheter 1E according to the sixth embodiment does not require the bifurcate connector 50 and the sealing portion 60 provided in the configuration described in the first embodiment. The catheter 1E according to the sixth embodiment includes a connector 50E instead of the connector 50 and a core-wire operating portion 90 instead of the sealing portion 60.

The connector 50E includes a hollow body portion 59E and a vane portion 51E. The inner cavity 51L of the body portion 59E serves as a guide-wire lumen and a thrombus lumen. The proximal end portion 20p of the hollow shaft 20 is joined to the distal end portion 59d of the body portion 59E. Moreover, the vane portion 51E is joined to the proximal end portion 591 of the body portion 59E. The vane portion 51E has a similar shape as the vane portion 51 according to the first embodiment, and the opening 51o at the proximal end side which leads to the inner cavity 51L is configured so that an aspirator can be attached. A material of the vane portion 51E may be similar to one used for the vane portion 51 according to the first embodiment. The vane portion 51E and the vane portion 51 may use the same material, or may use different materials.

The core-wire operating portion 90 includes a roller 91 arranged in the inner cavity 51L of the body portion 59E, and a handle 92 arranged at the outer side of the body portion 59E. The roller 91 and the handle 92 are connected via a revolving shaft, and the roller 91 rotates when the handle 92 is rotated in the direction of an arrow. The core wire 110 which is pre-wounded is fixed to the surface of the roller 91. Consequently, an operator rotates the handle 92 at the the core-wire operating portion 90 clockwise/counterclockwise instead of pulling/pushing the core wire 110 when operating the catheter 1E. The catheter 1E according to the sixth embodiment as described above can also demonstrate similar effects as the aforementioned first embodiment does. Moreover, according to the catheter 1E of the sixth embodiment, the catheter 1E can be configured using the connector 50E having no branched portion, leading to a compact configuration of the catheter 1E.

### Modified examples of the present embodiment

The present invention shall not be limited to the above embodiments, and can be implemented according to various aspects without departing from the scope and spirit of the present invention. For example, the following modified examples may be possible.

### Modified example 1

In the above first to sixth embodiments, the configurations of the catheter 1 and 1A to 1E are exemplified. However, various changes may be made in the configuration of the catheter 1. For example, a coating for improving antithrombogenicity, biocompatibility, lubricity, and wear-resistance and slideability may be applied on at least one of the inner peripheral surface and outer peripheral surface of each member of the catheter 1. For example, the connector 50 may be omitted. In this case, the vane portion 51, for example, may be attached to the proximal end portion 20p of the hollow shaft 20. For example, the marker 72 joined to the inner hollow shaft 100 may be omitted. Moreover, a separate marker other than the aforementioned marker 72 may be included.

### Modified example 2

In the above first to sixth embodiments, the configurations of the mesh members 10, 10A, and B are exemplified. However, various changes may be made in the configuration of the mesh member 10. For example, the mesh member 10 may formed by braiding the elemental wires 12 into a wave-like pattern or circular pattern instead of braiding the elemental wires 12 into a lattice-like pattern. For example, the element wires 12 may be formed of or coated with a radiopaque material in order to improve the visibility (the visibility particularly in the expanded state) of the mesh member 10.

For example, the mesh member 10 may be expanded and contracted by a method which does not use the core wire 110. In this case, the mesh member 10 may be formed of, for example, a shape memory alloy. Then, the mesh member 10 is covered with a substantially tubular sleeve for covering the mesh member 10 and maintaining the contracted state of the mesh member 10. An operating mechanism may be provided at the side of an operator, the operating mechanism being configured to enable the sleeve to be operated to cover or expose the mesh member 10. This can allow an operator to expand and contract the mesh member 10 without using the core wire 110. The substantially tubular sleeve and the operating machine described above correspond to the expanding and contracting functional portion.

### Modified example 3

The configurations of the catheters according to the first to sixth embodiments and the configurations of the catheters according to the modified examples 1 and 2 may be combined as appropriate. For example, the configuration according to the second and third embodiments where the film member is included (Figs. 7 and 8), or the configuration according to the fourth embodiment where the distal end tip 30 is not included (Fig. 9), or the configuration according to the fifth embodiment where the inner hollow shaft 100 is not included (Fig. 10) may be used in combination with the catheter 1E according to the sixth embodiment (Fig. 11)

As described above, the present aspect is described based on the embodiments and modified examples, but the aforementioned modes of implementing the aspect are provided merely for better understanding of the present aspect, and shall not be construed as limiting the present aspect. Alternations and improvements may be made in the present aspect without departing from the scope and spirit of the claims, and equivalents thereof fall within the scope of the present aspect. Moreover, a technical feature may be omitted, if desired, unless otherwise stated as essential in the present specification.

## Claims

1. A catheter (1; 1A; 1B; 1C; 1D; 1E) comprising:
a hollow shaft (20), and
a mesh member (10; 10A; 10B) joined to a distal end side of the hollow shaft (20) and capable of expanding and contracting in radial directions, **characterized by**
an expanding and contracting functional portion (110) for expanding and contracting the mesh member (10; 10A; 10B),
a distal-end fixed member (40; 40C) joined to a distal end side of the mesh member (10; 10A; 10B), and
an aspirator-attachment portion (50; 50E) for attachment of an aspirator, the aspirator-attachment portion (50; 50E) being provided at a proximal end side of the hollow shaft (20).

2. The catheter (1; 1A; 1B; 1C; 1D; 1E) according to claim 1, wherein
the catheter (1; 1A; 1B; 1C; 1D; 1E) is configured so that a target matter captured in an inner side of the mesh member (10; 10A; 10B) is directed toward the aspirator through the inside of the hollow shaft (20) by negative pressure applied in the inside of the hollow shaft (20) when the aspirator attached to the aspirator-attachment portion (50; 50E) is activated.

3. The catheter (1; 1A; 1B; 1C; 1D; 1E) according to claim 1 or 2, wherein
the expanding and contracting functional portion (110) is a core wire (110; 110E) having a distal end portion joined to the distal-end fixed member (40; 40C) and extending and passing through the insides of the mesh member (10; 10A; 10B) and the hollow shaft (20), and
the mesh member (10; 10A; 10B) is configured so that it expands due to compression along an axis-line direction by the distal-end fixed member (40; 40C) and the hollow shaft (20) when the core wire (110; 110E) is pulled along the axis-line direction from a proximal end side, and contracts due to extension along the axis-line direction by the distal-end fixed member (40; 40C) and the hollow shaft (20) when the core wire is pushed along the axis-line direction from the proximal end side.

4. The catheter (1A; 1B) according to any one of claims 1 to 3, further comprising
a first film member (81) having a film-like shape and covering at least a portion of a proximal end side of the mesh member (10A; 10B).

5. The catheter (1A) according to any one of claims 1 to 4, further comprising
a second film member (82) having a film-like shape and covering at least a portion of the distal end side of the mesh member (10A).

6. The catheter (1; 1A; 1B; 1C; 1E) according to any one of claims 1 to 5, further comprising
an inner hollow shaft (100) having a distal end portion joined to the distal-end fixed member (40; 40C) and forming a guide-wire lumen in the inside of the mesh member (10; 10A; 10B).
